# EUROPEAN PATENT SPECIFICATION

(11) **EP 0 555 007 B1**
(45) Date of publication and mention of the grant of the patent: **15.12.1999**
(21) Application number: 93300602.5
(22) Date of filing: 28.01.1993
(51) Int. Cl.: A61M 39/00, A61M 5/14, A61M 5/162

(54) **Drug delivery system**
Medikamentenabgabesystem
Système pour l'administration de médicament

(30) Priority: 30.01.1992 AU 61192
(43) Date of publication of application: 11.08.1993
(73) Proprietor: F.H. FAULDING & CO. LIMITED, Parkside South Australia 5063 (AU)
(72) Inventor: Rohrbough, John, Scottsdale Arizona 85258 (US)
(74) Representative: Williams, Janice

(56) References cited:
- WO-A-84/00893
- DE-U- 9 003 505
- FR-A- 2 200 022
- US-A- 3 994 296
- US-A- 4 559 038
- US-A- 4 657 486

## Description

The present invention relates to drug delivery systems, particularly systems for continuous delivery of drugs utilising pumps of the vacuum type.

It is known in the prior art to administer fluids to a patient in a number of ways. For example, a solution such as saline, glucose or an electrolyte in a glass or flexible container may be fed intravenously to a patient's venous access site via a length of flexible plastic tubing such a polyvinylchloride tubing. Flow from the container to the patient may be regulated by means of a roller clamp.

However, such gravity feed type systems are of limited application. This is particularly so where continuous or controlled application of drugs is required. For example, in the treatment of pain related conditions, it is known in the art to use a number of electronic pumping systems to deliver drugs to the patient. A number of positive pressure pumps, e.g. of the plunger type, are known in the field. An example thereof is the pump sold under the trade designation "Lifecare PCA+2 Infusion System" available from Abbott Laboratories. However, such positive feed systems suffer from a number of difficulties including the fact that with positive pressure systems, after the pump stops, contents may continue to be expelled or sucked back because of the positive pressure already created in the syringe or the like, thus making control less accurate. Further, with systems such as the Abbott Lifecare system, specially designed drug vials must be utilised.

It is also known in the prior art, for example via United States Patents 4,559,038 and 4,565,542, to utilise vacuum pump systems for the delivery of drugs. For example, a peristaltic pumping action is particularly well suited for the medical field. This is because peristaltic pumping action may be applied externally of the tubing carrying the intravenous or like fluid. Thus, sterile conditions of the fluid are maintained within the tubing, whilst imparting propulsion to the fluid. The peristaltic pumping action may also be applied to any point along the tubing. In a common type of peristaltic pump known in the prior art, a driving motor is connected to an array of cams angularly spaced from each other. The cams in turn drive cam followers connected to corresponding pressure fingers. These elements cooperate to impart a linear wave motion on the pressure fingers.

Whilst such vacuum systems provide significant advantages over the positive pressure systems know in the art, difficulties still remain with the peristaltic pump arrangements. For example, containers utilised with the peristaltic systems are normally empty bags which must be filled with a medicament by the operator. Such containers are inconvenient to fill, difficult to use, and increase the danger of inadvertent spillage or contamination. Alternatively where the medicament is provided in a disposable cassette form, the cassettes are very expensive to replace.

United States Patent No. 3,994,296 discloses a syringe in which the needle is mounted in an inner tubular member which terminates in a connector for connection to a stopper piston of a medicament vial. When assembled, one ad of the needle penetrates the stopper piston, so that as the vial is pressed towards the syringe, the stopper acts as a piston forcing liquid from the vial through the needle. A similar syringe construction is also shown in FR-A-2200022.

Reference is also made to US-A-4657486 which describes a portable infusion device for injecting medical fluids into a human or animal body by means of a positive pressure pump that is automatically operated at selected tune intervals to inject accurate amounts of fluid medicine into the body.

It is an object of the present invention to overcome, or at least alleviate, one or more of the difficulties or deficiencies related to the prior art.

According to one aspect of the present invention there is provided:
an assembly for use with pumping means to form drug delivery apparatus for use with a medicament vial which is closed at one end by a penetrable plunger movable into the vial to reduce the interior volume by an amount corresponding to the medicament volume drawn out from the vial, the assembly comprising:
a housing;
a predetermined vial receiving region within the housing having a predetermined length, for receiving a said vial within the housing;
an adapter having a connection socket for connection to the plunger of a said vial, a connection nozzle for connection to tube means leading from the adapter, and a tubular spike for penetrating the plunger when the plunger is connected to the connection socket to thereby flow connect the vial with a fluid pathway extending through the adapter;
   characterised in that:
the housing comprises a pressure plate defining an interface for a said pumping means; and
   the assembly further comprises: mounting means for mounting a said vial in a substantially fixed position in the vial receiving region within the housing;
flexible tube means having an end coupled to the connection nozzle, a peristaltically pumpable first portion supported on the pressure plate in a substantially fixed position relative to the housing for communicating with the pumping means when the housing is coupled thereto, and a coil portion between said end and said pumpable portion to permit the adapter to move relative to the housing substantially along the length of the vial receiving region, whereby, in use, when the assembly is coupled to a said pumping means and to a said vial, the coil portion of the tube means permits the adapter to move with the plunger of the vial into the vial as the medicament is drawn out by the suction generated in the tube means by the pumping means.

In a second aspect, the invention provides drug delivery apparatus for use with a medicament vial which is closed at one end by a penetrable plunger movable into the vial to reduce the interior volume by an amount corresponding to the medicament volume drawn out from the vial, the apparatus being operable to deliver the medicament from the vial and comprising:
a housing;
a predetermined vial receiving region within the housing having a predetermined length for receiving a said vial within the housing;
an adapter having a connection socket for connection to the plunger of a said vial, a connection nozzle for connection to a tube means leading from the adapter, and a tubular spike for penetrating the plunger when the plunger is connected to the connection socket to thereby flow connect the vial with a fluid pathway extending through the adapter; characterised by:
mounting means for mounting a said vial in a substantially fixed position in the vial receiving region within the housing;
flexible tube means having an end coupled to the connection nozzle, a first portion retained in a substantially fixed position relative to the housing for communicating with a pumping means, and a second coil portion between said end and said first portion to accommodate the movement of the adapter relative to the housing substantially along the length of the vial receiving region; and
pumping means communicating with the first portion of the tube means to effect pumping thereof by applying pressure to the exterior of the tube means so as to propel medicament along the tube means;
whereby, in use, the coil portion permits the adapter to move with the plunger of a said vial into the vial as the medicament is drawn out by the action of the pumping means on the tube means.

If desired, the tube means, or tubing, may be connected at its inner end to a plurality of adaptor-vial units thus increasing the capacity of the suction delivery device.

It will be understood that a suction delivery device embodying the present invention may function as follows:

After the adaptor is connected to the medicament vial and the spike penetrates the penetrable plunger, the tubing may then be primed. This may be achieved by standing the vial on its base and depressing the adaptor, thus causing the fluid to flow into the tubing and priming the device. When the pumping means is turned on, fluid is drawn from the container and the vacuum created by the fluid removal causes the plunger to progress towards the vial base. The plunger is able to move along the vial because the priming operation breaks the initial friction set between the plunger and vial.

The suction pumping operation of the device will cause liquid transfer from the medicament vial via the adaptor to the patient, and simultaneously compensating vial movement longitudinally relative to the penetrable plunger under external atmospheric pressure, reduces the chamber volume by an amount generally equal to the liquid transfer.

The suction delivery device may include a cylindrical medicament vial. The cylindrical medicament vial may be of standard known type. The cylindrical medicament vial may be of the shell glass vial type. The vial may include a pharmaceutical product. The pharmaceutical product may be of any suitable type. The pharmaceutical may include a narcotic drug. The narcotics included may be selected from one or more of morphine, meperidine, fentanyl, hydromorphone; and salts thereof and the like.

The pharmaceutical may include an anti-cancer formulation. The anti-cancer formulation may be selected from one or more of anthracyclines, such as doxorubicin, cytarabine, vinblastine, cisplatin, bleomycin, mitomycin; and salts thereof, and the like.

The adaptor in embodiments of the present invention may be fabricated from conventional materials and components. A plastics material may be used for the general construction thereof. An injection moulding construction may be used.

The tubular spike or needle may be formed in metal, for example stainless steel.

The adaptor may be of generally cylindrical shape. The adaptor may include a generally radial front wall forming its closed end. The second connection means may be a connection nozzle which may be of generally cylindrical shape. The connection nozzle may taper towards its outer end.

The first connection means may be a connection socket defining an axial cylindrical recess and the tubular spike may define an axial hollow cylindrical tubular portion centrally arranged coaxially with the cylindrical recess.

The connection socket may further include an internal thread connection formation adapted, in use, to mate with a counterpart external thread connection formation on the penetrable plunger of a corresponding vial to be connected thereto.

The tubular spike may include a pointed free end and a base end mounted centrally within the front wall of the adaptor.

The flexible tubing of the suction delivery device may be of any suitable type. The tubing may be of the non-collapsible type. The tubing may be selected to have a low priming volume. The tubing may be of the non-kink, non-collapsible type. The tubing may be of the non-kink microbore type.

In a preferred embodiment, the tubing may include a section in the region of the pumping means to permit accurate fluid delivery to the patient. The tubing may include a section formed from a silicone type material, at least in the region of the pumping means. A silastic type material is preferred.

In a further preferred embodiment, the tubing of the suction delivery device may include a coiled section proximate the inner end thereof.

The coiled section may assist in the assembly of the component part of the suction delivery device, The coiled section may further reduce the likelihood of collapse of the flexible tubing during use. In use, the coiled tubing uncoils and allows the plunger to move freely towards the closed end of the vial.

The flexible tubing may be connected to the connection nozzle of the adaptor in fluid tight manner. The flexible tubing may overlay the connection nozzle of the adaptor. Alternatively the inner open end of the tubing terminates in a connection nozzle counterpart to the connection nozzle of the adaptor.

The counterpart connection nozzle may include a sleeve section adapted to receive the inner open end of the tubing. The sleeve may be sized so that the tubing is compressed therewithin to provide a fluid tight fit.

More preferably the connection nozzle on the adaptor has a luer lock connector to mate with a counterpart luer lock connector on the counterpart connection nozzle on the inner end of the length of tubing.

Accordingly in this embodiment the suction delivery device may further include a sealing means connecting the inner end of the flexible tubing to the connection nozzle of the adaptor.

The sealing means may be a clamp or like arrangement. A sealing ring may be used. A flexible sealing ring, for example of the O-ring type, may be used. The sealing ring may be formed of an elastomeric material.

The pumping means used in connection with the suction delivery device may be of any suitable type. A peristaltic pump may be used. A linear peristaltic pump may be used. A peristaltic pump sold under the trade designation Deltec Cadd-PCA and available from Pharmacia has been found to be suitable.

It will be understood the suction delivery apparatus device is particularly suitable for Patient Controlled Analgesia (PCA) and like applications. Alternatively, continuous delivery or pulsed delivery may also be suitable.

The suction delivery device may be provided in a ready-to-use kit form. The kit may include one or more adaptors as described above, one or more lengths of tubing, one or more filled cylindrical medicament vials and a housing incorporating the tubing arrangements described above. Such kits are particularly suitable for use both in hospital and home treatment environments for intravenous, subcutaneous or epidural drug delivery.

The kits embodying the present invention have the added advantage that standard medicament vials may be utilised therewith.

As described above, the suction delivery device, in use, requires a priming step to initiate product delivery. This may be achieved by standing the vial on its base and depressing the adaptor. In order to assist in the priming step the adaptor may include at least one finger tab on the outer surface thereof.

Preferably the adaptor includes a pair of finger tabs mounted on opposite sides of the adaptor. The finger tab or tabs may be formed integrally with the adaptor.

In a preferred embodiment two or more adaptor-vial units may be included in the suction delivery device. This increases the capacity of the device which may be important in continuous infusion applications. In such embodiments, the lengths of tubing extending from each unit may be joined in a single length of tubing prior to contact with the pumping means.

In a preferred form, the suction delivery device may include
a junction member having first and second inlet arms communicating with an outlet arm;
the first inlet arm arranged, in use, to flow connect with a first length of tubing connected to a first adaptor;
the second inlet arm arranged, in use, to flow connect with a second length of tubing connected to a second adaptor;
a third length of tubing having an inner end and outer end, the inner end arranged, in use, to flow connect with the outlet arm of the junction member; the suction delivery device being arranged in use to coact with means for applying a pumping action on the exterior of the third length of tubing to provide fluid propulsion intermediate the inner and outer end thereof.

The junction member may be a Y-shaped or T-shaped junction. The junction member may include sealing means for sealing connection to the respective lengths of tubing.

It has been found that by attaching a pair of cylindrical medicament vials in parallel, the linear peristaltic action of the pump creates a vacuum in the tubing which sucks fluid from either of the vials. Accordingly, if one vial empties before the other, the pump will automatically draw fluid from the other vial.

The housing of the suction delivery device may be connected to any portion of the pumping unit, in use. The housing of the suction delivery device may be adapted for snap-fit connection to the pumping unit. Alternatively the housing may be removably hinged to the pumping unit at one end and held by a locking mechanism at the other end. The housing may be formed of a plastics material. The housing may also function to provide containment of fluid should the cylindrical medicament vial or vials suffer a breakage.

The housing may have associated with it a pressure plate, the housing being attached to the pressure plate. The pressure plate is adapted to interface with the pump body, and connect thereto. The pressure plate may connect to the pump body by known means, for example by hooks or other locking mechanisms, The tubing is positioned to run across the face of the pressure plate where the peristaltic action of the pump is able to act upon the tubing.

Preferably the housing includes a hollow body having a closed inner end and an open outer end and including a base wall defining the closed inner end and a connection formation proximate the outer end thereof.

The housing may include at least one mounting means within the hollow body arranged to releasably hold at least one cylindrical medicament vial. One or more clamping means may be used. A biased clamp is preferred.

In an alternative embodiment, the housing of the suction delivery device may include a hollow longitudinal outer shell releasably connected to the base wall of the hollow body, and having a front closed end and a rear open end, the hollow outer shall being adapted to receive and enclose a cylindrical medicament vial.

The hollow longitudinal outer shell may function to protect the cylindrical medicament vial and, when removed, may provide for easy replacement of an empty medicament vial. The hollow longitudinal outer shell may be connected to the base wall of the hollow body in any suitable manner. A luer lock or like connection may be used.

In this form, the base wall of the hollow body may include a central recess adapted to receive a portion of the connection nozzle of the adaptor and counterpart connection nozzle on the inner end of the tubing.

The length of the hollow longitudinal outer shell generally corresponds to the vial length so that the position of the vial is substantially fixed in the housing. Vials of different lengths may be used within the housing by having several interchangeable hollow longitudinal outer shells of differing lengths, and sufficient tube lengths. On the other hand, in an alternative embodiment, the hollow longitudinal outer shell may contain spring means that act on the base of the vial, whereby vials of different lengths may be accommodated in the hollow longitudinal outer shell with stability.

It is desirable that the housing be et approximately equal pressure both internally and externally. Thus the housing may further include a venting means. A hydrophobic filter vent may be included.

If desired the suction delivery device may be provided in a ready-to-use form. Accordingly, the device may include a pumping unit.

Embodiments of the present invention will now be more fully described with reference to the accompanying drawings. It should be understood, however, that the description following is illustrative only and should not be taken in any way as a restriction on the scope of the invention.

In the figures,
Figure 1 is a cross-sectional stylised view of a suction delivery device embodying the present invention.
Figure 2 is a further embodiment of a suction delivery device according to the present invention in which a pair of medicament vials are utilised.

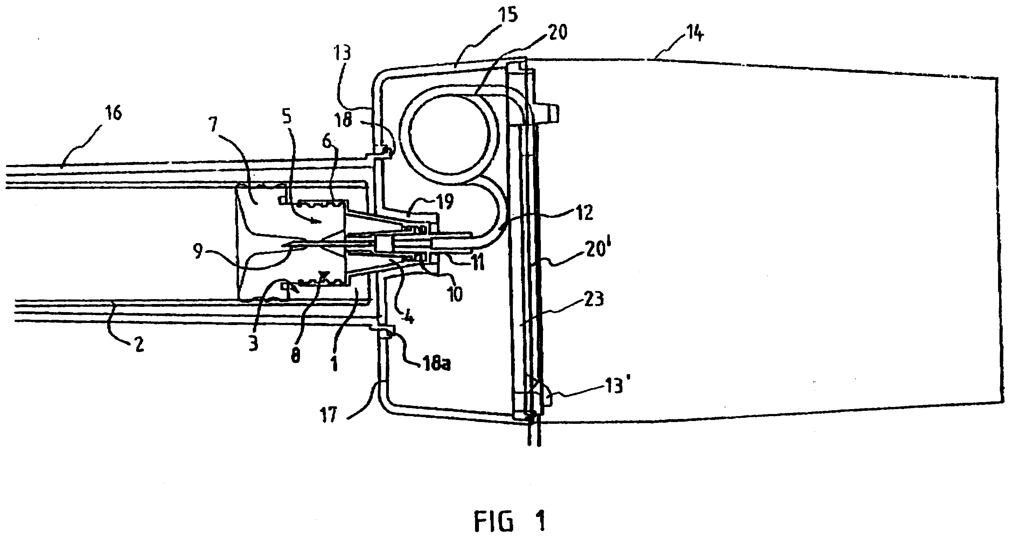

In more detail, Figure 1 illustrates on adaptor 1 adapted for connection to a shell glass vial 2. The adaptor 1 has an open inner end 3 and closed outer end 4. The open inner end 3 includes a connection socket 5 which is provided with an internal thread 6. In use, the adaptor 1 is connected to a penetrable plunger 7 of the shell glass vial 2. The penetrable plunger includes an external threaded section 8 screwed into the connection socket 5 of adaptor 1. The screwing motion permits the needle 9 of adaptor 1 to penetrate the penetrable plunger to flow connect the interior of the shell glass vial with the interior of the needle.

The outer end 4 of adaptor 1 includes a connection nozzle 10 connected to a counterpart connection nozzle 11, for example via a luer lock arrangement, on the end of a length of tubing 12. This connects the interior of the shell glass vial to the interior of the length of tubing via the fluid pathway defined by needle 9.

The suction delivery device includes a housing 13, which may be adapted for removable hinged connection to a pump body 14. The housing 13 includes a hinge member 13' and a latch member (not shown) which permit the housing to be locked into the base of the pump body via counterpart locking means (not shown).

The housing also includes a pressure plate 23 which interfaces with the pump body 14.

The housing 13 includes a hollow body 15 and hollow outer shell 16. The hollow outer shell 16 is releasably connected to the base wall 17 of the hollow body via luer lock tabs 18 and 18a. The shell glass vial 2 is surrounded by the hollow outer shell 16. The base wall 17 has a central recess 19 which receives the connecting portion of the adaptor 1 and counterpart connection nozzle 11.

The length of tubing 12 includes a coiled section 20 which may uncoil as the fluid contents are withdrawn from the shell glass vial 2. This assists in preventing collapse of the tubing during use and permits easy replacement of empty vials. The length of tubing 12 further includes a section of non-collapsible tubing 20' which runs along the top of pressure plate 23 and upon which the pumping action of the pump 14 is applied via reciprocating cams (not shown).

The drug delivery system embodying the invention is gravity independent and therefore will operate satisfactorily regardless of orientation. The position of the pump body 14 relative to the housing 13 is not critical. As illustrated, the pump body 14 is above the housing 13, however the positions may be reversed. Also, the housing 13 may be alongside, in front of, behind, etc., the pump body in alternative embodiments in accordance with the invention.

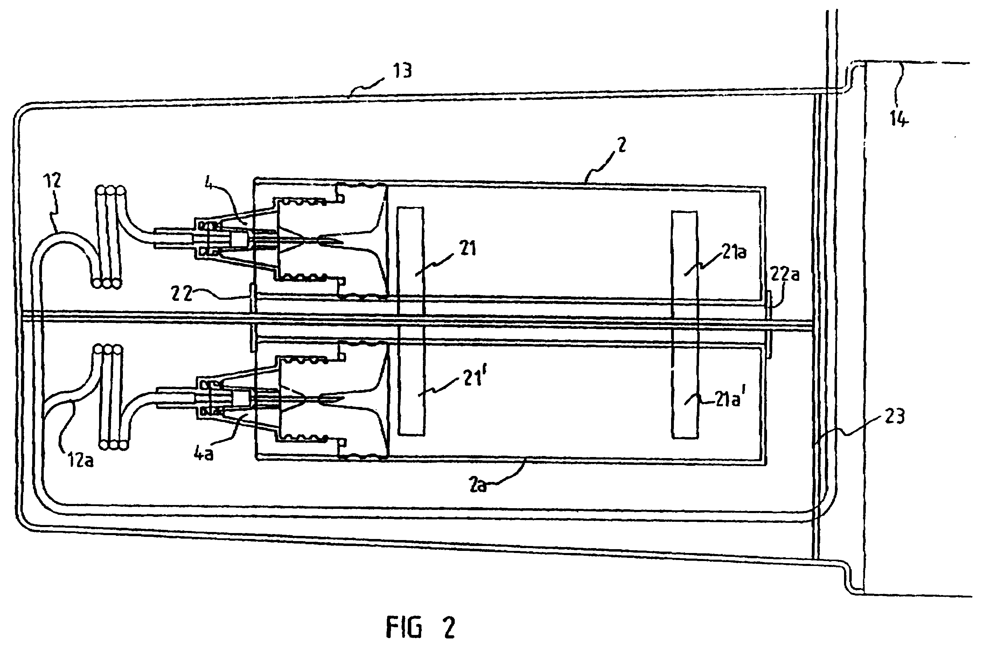

In an alternative embodiment, as illustrated in Figure 2, the suction delivery device may include a pair of shell glass vials 2 and 2a connected by a pair of adaptors 4 and 4a which are in turn connected to lengths of tubing 12 and 12a. The tubing 12 and 12a are joined via a Y-shaped junction member (not shown). Each shell glass vial 2, 2a is mounted within the housing 13 on support clips 21, 21a and 21' and 21a' respectively, between detent means 22 and 22a.

In this embodiment the housing 13 completely encloses the suction delivery device. Replacement of empty shell glass vials is achieved by removal of the housing from the pump body 14. The housing is then opened and the empty shell glass vials 2 and 2a detached from the adaptors 4 and 4a and replaced.

In order to ensure a fluid tight seal, the tubing may be sealed via an O-ring at the point where the tubing exits the pump body.

Finally, it is to be understood that various other modifications and/or alterations may be made. For example, in the figures, the cylindrical medicament vials are shown in vertical orientation. The cylindrical medicament vials may be mounted horizontally if desired.

## Claims

1. An assembly for use with pumping means (14) to form drug delivery apparatus for use with a medicament vial (2) which is closed at one end by a penetrable plunger (7) movable into the vial to reduce the interior volume by an amount corresponding to the medicament volume drawn out from the vial, the assembly comprising:
a housing (13);
a predetermined vial receiving region within the housing having a predetermined length, for receiving a said vial within the housing;
an adapter (1,4) having a connection socket (5) for connection to the plunger (7) of a said vial, a connection nozzle (10) for connection to tube means (12) leading from the adapter, and a tubular spike (9) for penetrating the plunger (7) when the plunger is connected to the connection socket (5) to thereby flow connect the vial (2) with a fluid pathway extending through the adapter;
characterised in that:
the housing (13) comprises a pressure plate (23) defining a interface for a said pumping means;
and the assembly further comprises:
mounting means (16; 21, 21a) for mounting a said vial (2) in a substantially fixed position in the vial receiving region within the housing;
flexible tube means (12) having an end coupled to the connection nozzle (10), a peristaltically pumpable first portion (20') supported on the pressure plate in a substantially fixed position relative to the housing (13) for communicating with the pumping means (14) when the housing is coupled thereto, and a coil portion (20) between said end and said pumpable portion (20') to permit the adapter to move relative to the housing (13) substantially along the length of the vial receiving region, whereby, in use, when the assembly is coupled to a said pumping means (14) and to a said vial (2), the coil portion (20) of the tube means permits the adapter (1,4) to move with the plunger (7) of the vial into the vial as the medicament is drawn out by the suction generated in the tube means by the pumping means.

2. An assembly according to claim 1, wherein the housing (13) comprises a hollow body (15) ad a hollow longitudinal outer shell (16) releasably connected to a base wall (17) of the hollow body, and having a closed end ad an open end, the hollow outer shell being configured to receive and enclose a said medicament vial.

3. An assembly according to claim 1, wherein the mounting means comprises a clamp for releasably holding a said vial.

4. An assembly according to claim 1, 2 or 3, further comprising a second vial adapter (1a) for coupling to the plunger of a further medicament vial, the tube means further comprising a junction between the first and second portions (20', 20) of tube, and a third tube portion (20a) extending from the junction to the second adapter to enable medicament delivery in parallel from two vials.

5. Drug delivery apparatus for use with a medicament vial which is closed at one end by a penetrable plunger movable into the vial to reduce the interior volume by an amount corresponding to the medicament volume drawn out from the vial, the apparatus being operable to deliver the medicament from the vial and comprising:
a housing (13);
a predetermined vial receiving region within the housing having a predetermined length for receiving a said vial within the housing;
an adapter (1, 4) having a connection socket (5) for connection to the plunger (7) of a said vial, a connection nozzle (10) for connection to a tube means (12) leading from the adapter, and a tubular spike (9) for penetrating the plunger when the plunger (7) is connected to the connection socket (5) to thereby flow connect the vial (2) with a fluid pathway extending through the adapter;
characterised by:
mounting means (16; 21,21a) for mounting a said vial (2) in a substantially fixed position in the vial receiving region within the housing (13);
flexible tube means (12) having an end coupled to the connection nozzle (10), a first portion (20') retained in a substantially fixed position relative to the housing for communicating with a pumping means, and a second coil portion (20) between said end and said first portion (20') to accommodate the movement of the adapter (1,4) relative to the housing substantially along the length of the vial receiving region; and
pumping means (14) communicating with the first portion (20') of the tube means to effect pumping thereof by applying pressure to the exterior of the tube means so as to propel medicament along the tube means;
whereby, in use, the coil portion (20) permits the adapter (1,4) to move with the plunger (7) of a said vial (2) into the vial as the medicament is drawn out by the action of the pumping means (14) on the tube means.

6. Apparatus according to the claim 5, wherein the tubular spike includes a pointed free end and a base end mounted centrally within the socket.

7. Apparatus according to claim 6, wherein the socket comprises a cylindrical recess (5), the socket further comprising an inwardly facing screw thread for threadedly engaging the portion of the plunger (7) to be received in the recess in use.

8. Apparatus according to any of claims 5 to 7, wherein the housing (13) is releasably attached to the pumping means (14).

9. Apparatus according to any of claims 5 to 8, wherein the housing comprises a pressure plate on which the first portion of the tube is supported, the pressure plate forming an interface between the housing and the pumping means.

10. Apparatus according to claim 8 or 9, wherein the housing (13) comprises a hollow body (15) and a hollow longitudinal outer shell (16) releasably connected to a base wall (17) of the hollow body, and having a closed end and an open end, the hollow outer shell being configured to receive and enclose a said medicament vial.

11. Apparatus according to any of claims 5 to 9, wherein the means for mounting the vial comprises a clamp (21,21a).

12. Apparatus according to of claims 5 to 11, wherein the pumping means comprises a peristaltic pump (14) acting lengthwise on said first portion (20') of the tube means to produce a linear pumping action thereon.

13. Apparatus according to any of claims 5 to 12, further comprising a second vial adapter (1a) for coupling to the plunger of a further medicament vial, the tube further comprising a junction between the first and second portions (20', 20) of tube, and a third tube portion (20a) extending from the junction to the second adapter to enable medicament delivery in parallel from two vials.

14. A drug delivery system comprising an apparatus as defined in any of claims 5 to 13, and a medicament vial (2) which is closed at one end by a penetrable plunger (7) movable into the vial to reduce the interior volume by an amount corresponding to the medicament volume drawn out from the vial, the plunger being connected to the connection socket of the adapter.

15. A drug delivery system comprising apparatus as defined in claim 13, and first and second medicament vials (2, 2a) each of which is closed at one end by a penetrable plunger (7) connected to a respective one of the adapters, each plunger being movable into the respective vial to reduce the interior volume by an amount corresponding to the medicament volume drawn out from the vial.

## Patentansprüche

1. Aufbau zum Gebrauch mit Pumpmitteln (14), um eine Medikamentenabgabevorrichtung für den Gebrauch mit einem Medikamentenfläschchen (2) zu bilden, das an einem Ende durch einen durchdringbaren Kolben (7) verschlossen ist, der in das Fläschchen bewegbar ist, um das innere Volumen um einen Betrag zu reduzieren, welcher dem aus dem Fläschchen gezogenen Medikamentenvolumen entspricht, wobei der Aufbau aufweist: ein Gehäuse (13);
einen vorbestimmten Fläschchenaufnahmebereich innerhalb des Gehäuses mit einer vorbestimmten Länge zur Aufnahme des Fläschchens innerhalb des Gehäuses;
einen Adapter (1, 4) mit einer Verbindungsmuffe (5) für die Verbindung mit dem Kolben (7) des Fläschchens, einer Verbindungsdüse (10) für die Verbindung mit von dem Adapter führenden Rohrmitteln (12) und einer rohrförmigen Spitze (9) zum Durchdringen des Kolbens (7), wenn der Kolben mit der Verbindungsmuffe (5) verbunden ist, um dadurch das Fläschchen (2) mit einem Fluidweg in Fließverbindung zu bringen, der sich durch den Adapter erstreckt;
dadurch gekennzeichnet, daß:
das Gehäuse (13) eine Druckplatte (23) aufweist, die eine Grenzfläche für ein Pumpenmittel bestimmt;
und der Aufbau ferner aufweist:
Befestigungsmittel (16; 21, 21a) für die Befestigung eines Fläschchens (2) in einer im wesentlichen festen Position in dem Fläschchenaufnahmebereich innerhalb des Gehäuses;
flexible Rohrmittel (12), deren eines Ende mit der Verbindungsdüse (10) gekoppelt ist, einen peristaltisch pumpbaren ersten Abschnitt (20'), der auf der Druckplatte in einer im wesentlichen festen Position bezüglich des Gehäuses (13) gestützt wird, um mit den Pumpmitteln (14) in Verbindung zu stehen, wenn das Gehäuse daran gekoppelt ist, und einen Spulenabschnitt (20) zwischen dem Ende und dem pumpbaren Abschnitt 20', um dem Adapter zu erlauben, sich bezüglich des Gehäuses (13) im wesentlichen entlang der Länge des Fläschchenaufnahmebereichs zu bewegen, wodurch im Gebrauch, wenn der Aufbau an die Pumpmittel (14) und das Fläschchen (2) gekoppelt ist, der Spulenabschnitt (20) der Rohrmittel dem Adapter (1, 4) erlaubt, sich mit dem Kolben (7) des Fläschchens in das Fläschchen zu bewegen, wenn das Medikament durch das Saugen herausgezogen wird, welches in den Rohrmitteln durch die Pumpmittel erzeugt wird.

2. Aufbau nach Anspruch 1, wobei das Gehäuse (13) einen hohlen Körper (15) und einen hohlen, länglichen äußeren Mantel (16) aufweist, der lösbar mit einer Basiswand (17) des hohen Körpers verbunden ist und ein geschlossenes und ein offenes Ende hat, wobei der hohle äußere Mantel aufgebaut ist, um das Medikamentenfläschchen aufzunehmen und einzuschließen.

3. Aufbau nach Anspruch 1, wobei das Befestigungsmittel eine Klammer zum lösbaren Halten des Fläschchens aufweist.

4. Aufbau nach Anspruch 1, 2 oder 3, der ferner einen zweiten Fläschchenadapter (1a) für die Kupplung an den Kolben eines weiteren Medikamentenfläschchens aufweist, wobei die Rohrmittel ferner eine Verbindung zwischen den ersten und zweiten Abschnitten (20', 20) des Rohres aufweisen und sich ein dritter Rohrabschnitt (20a) von der Verbindung zu dem zweiten Adapter erstreckt, um Medikamentenabgabe parallel aus zwei Fläschchen zu ermöglichen.

5. Medikamentenabgabevorrichtung für den Gebrauch mit einem Medikamentenfläschchen, das an einem Ende durch einen durchdringbaren Kolben geschlossen ist, der in das Fläschchen bewegbar ist, um das innere Volumen um einen Betrag zu reduzieren, welcher dem aus dem Fläschchen gezogenen Medikamentenvolumen entspricht, wobei die Vorrichtung betriebsfähig ist, das Medikament aus dem Fläschchen abzugeben, und aufweist:
ein Gehäuse (13);
einen vorbestimmten Fläschchenaufnahmebereich innerhalb des Gehäuses mit einer vorbestimmten Länge für die Aufnahme des Fläschchens innerhalb des Gehäuses;
einen Adapter (1, 4) mit einer Verbindungsmuffe (5) für die Verbindung mit dem Kolben (7) des Fläschchens, eine Verbindungsdüse (10) für die Verbindung mit einem von dem Adapter führenden Rohrmittel (12) und eine rohrförmige Spitze (9), um den Kolben zu durchdringen, wenn der Kolben (7) mit der Verbindungsmuffe (5) verbunden ist, um dadurch das Fläschchen (2) mit einem Fluidweg in Fließverbindung zu bringen, der sich durch den Adapter erstreckt;
gekennzeichnet durch:
Befestigungsmittel (16; 21, 21a) für die Befestigung des Fläschchens (2) in einer im wesentlichen festen Position in dem Fläschchenaufnahmebereich innerhalb des Gehäuses (13);
flexible Rohrmittel (12) mit einem Ende das an die Verbindungsdüse (10) gekoppelt ist, einem ersten Abschnitt (20'), der in einer im wesentlichen festen Position bezüglich des Gehäuses zurückgehalten ist, um mit einem Pumpmittel in Verbindung zu stehen, und einem zweiten Spulenabschnitt (20) zwischen dem Ende und dem ersten Abschnitt (20'), um die Bewegung des Adapters (1, 4) bezüglich des Gehäuses im wesentlichen entlang der Länge des Fläschchenaufnahmebereichs anzupassen; und
Pumpmittel (14), die mit dem ersten Abschnitt(20') der Rohrmittel in Verbindung stehen, um deren Pumpen durch Aufbringen von Druck auf das Äußere der Rohrmittel zu bewirken, um Medikament entlang der Rohrmittel vorwärts zu treiben;
wodurch in Gebrauch der Spulenabschnitt (20) dem Adapter (1, 4) erlaubt, sich mit dem Kolben (7) des Fläschchens (2) in das Fläschchen zu bewegen, wenn Medikament durch die Tätigkeit der Pumpmittel (14) an den Rohrmitteln herausgezogen wird.

6. Vorrichtung nach Anspruch 5, wobei die rohrförmige Spitze ein zugespitztes freies Ende und ein Basisende aufweist, das zentral innerhalb der Muffe befestigt ist.

7. Vorrichtung nach Anspruch 6, wobei die Muffe eine zylindrische Ausnehmung (5) aufweist, die Muffe ferner ein nach innen gekehrtes Schraubengewinde aufweist, um mit dem Teil des Kolbens (7) in Gewindeeingriff zu kommen, der bei Gebrauch in der Ausnehmung aufgenommen werden soll.

8. Vorrichtung nach einem der Ansprüche 5 bis 7, wobei das Gehäuse (13) lösbar an den Pumpmitteln (14) befestigt ist.

9. Vorrichtung nach einem der Ansprüche 5 bis 8, wobei das Gehäuse eine Druckplatte aufweist, auf welcher der erste Abschnitt des Rohres gestützt ist, und die Druckplatte eine Grenzfläche zwischen dem Gehäuse und dem Pumpmittel bildet.

10. Vorrichtung nach Anspruch 8 oder 9, wobei das Gehäuse (13) einen hohlen Körper (15) und einen hohen, länglichen Außenmantel (16) aufweist, der lösbar mit einer Basiswand (17) des hohlen Körpers verbunden ist, und ein geschlossenes und ein offenes Ende hat, wobei der hohle Außenmantel so aufgebaut ist, daß er das Medikamentenfläschchen aufnimmt und einschließt.

11. Vorrichtung nach einem der Ansprüche 5 bis 9, wobei das Mittel zur Befestigung des Fläschchens eine Klammer (21, 21a) aufweist.

12. Vorrichtung nach einem der Ansprüche 5 bis 11 wobei das Pumpmittel eine peristaltische Pumpe (14) aufweist, die längs auf dem ersten Abschnitt (20') des Rohrmittels arbeitet, um auf diesem eine lineare Pumptätigkeit herzustellen.

13. Vorrichtung nach einem der Ansprüche 5 bis 12, ferner mit einem zweiten Fläschchenadapter (1a) zum Koppeln an den Kolben eines weiteren Medikamentenfläschchens, wobei das Rohr ferner eine Verbindung zwischen den ersten und zweiten Abschnitten (20', 20) des Rohres aufweist und ein dritter Rohrabschnitt (20a) sich von der Verbindung zu dem zweiten Adapter erstreckt, um die Medikamentenabgabe parallel von zwei Fläschchen zu ermöglichen.

14. Medikamentenabgabesystem mit einer Vorrichtung nach einem der Ansprüche 5 bis 13 und einem Medikamentenfläschchen (2), das an einem Ende durch einen durchdringbaren Kolben (7) geschlossen ist, der in das Fläschchen bewegbar ist, um das innere Volumen um einen Betrag zu reduzieren, welcher dem Medikamentenvolumen entspricht, das aus dem Fläschchen gezogen wurde, wobei der Kolben mit der Verbindungsmuffe des Adapters verbunden ist.

15. Medikamentenabgabesystem mit einer Vorrichtung nach Anspruch 13 und ersten und zweiten Medikamentenfläschchen (2, 2a), von denen jedes an einem Ende durch einen durchdringbaren Kolben (7) verschlossen ist, der mit einem entsprechenden Adapter verbunden ist, wobei jeder Kolben in das entsprechende Fläschchen bewegbar ist, um das innere Volumen um einen Betrag zu reduzieren, welcher dem Medikamentenvolumen entspricht, das aus dem Fläschchen gezogen wurde.

## Revendications

1. Agencement pour utilisation avec un moyen de pompage (14) pour former un appareil pour dispenser des médicaments, destiné à être utilisé avec un flacon de médicament (2) qui est fermé à une extrémité par un plongeur pénétrable (7) adapté à se déplacer dans le flacon pour diminuer le volume interne d'une quantité correspondant au volume de médicament extrait du flacon, l'agencement comprenant :
un boîtier (13) ;
une région de réception de flacon prédéterminée au sein du boîtier ayant une longueur prédéterminée, pour recevoir un dit flacon au sein du boîtier ;
un adaptateur (1, 4) comportant une douille de connexion (5) pour connexion au plongeur (7) d'un dit flacon, une buse de connexion (10) pour connexion à un moyen de tube (12) partant de l'adaptateur, et une pointe tubulaire (9) pour pénétrer dans le plongeur (7) lorsque le plongeur est relié à la douille de connexion (5) de manière à relier en écoulement le flacon (2) avec un passage de fluide s'étendant à travers l'adaptateur ;
caractérisé en ce que :
le boîtier (13) comprend une plaque de pression (23) définissant une interface pour un dit moyen de pompage ;
et l'agencement comprend également :
un moyen de montage (16 ; 21, 21a) pour monter un dit flacon (2) dans une position sensiblement fixe dans la région de réception de flacon au sein du boîtier ;
un moyen de tube flexible (12) comportant une extrémité couplée à la buse de connexion (10), une première portion susceptible d'un pompage péristaltique (20') supportée sur la plaque de pression dans une position sensiblement fixe relativement au boîtier (13) pour être en communication avec le moyen de pompage (14) lorsque le boîtier est couplé à celui-ci, et une portion en serpentin (20) entre ladite extrémité et ladite portion susceptible d'un pompage (20') afin de permettre à l'adaptateur de se déplacer relativement au boîtier (13) sensiblement le long de la région de réception de flacon, de manière que, à l'utilisation, lorsque l'agencement est couplé à un dit moyen de pompage (14) et à un dit flacon (2), la portion en serpentin (20) du moyen de tube permette à l'adaptateur (1, 4) de se déplacer avec le plongeur (7) du flacon dans le flacon tandis que le médicament est extrait par l'aspiration produite dans le moyen de tube par le moyen de pompage.

2. Agencement selon la revendication 1, dans lequel le boitier (13) comprend un corps creux (15) et une enveloppe externe longitudinale creuse (16) reliée avec possibilité de séparation à une paroi de base (17) du corps creux, et comportant une extrémité fermée et une extrémité ouverte, l'enveloppe externe creuse étant configurée de manière à recevoir et enfermer un dit flacon de médicament.

3. Agencement selon la revendication 1, dans lequel le moyen de montage comprend une attache pour maintenir avec possibilité de séparation un dit flacon.

4. Agencement selon la revendication 1, 2 ou 3, comprenant également un second adaptateur de flacon (1a) destiné à être couplé au plongeur d'un autre flacon de médicament, le moyen de tube comprenant également une jonction entre les première et deuxième portions (20', 20) de tube, et une troisième portion de tube (20a) s'étendant de la jonction au second adaptateur pour permettre de dispenser un médicament en parallèle depuis deux flacons.

5. Appareil pour dispenser des médicaments, destiné à être utilisé avec un flacon de médicament qui est fermé à une extrémité par un plongeur pénétrable adapté à se déplacer dans le flacon pour diminuer le volume interne d'une quantité correspondant au volume de médicament extrait du flacon, l'appareil étant adapté à dispenser le médicament depuis le flacon et comprenant :
un boitier (13) ;
une région de réception de flacon prédéterminée au sein du boîtier ayant une longueur prédéterminée pour recevoir un dit flacon au sein du boîtier ;
un adaptateur (1, 4) comportant une douille de connexion (5) pour connexion au plongeur (7) d'un dit flacon, une buse de connexion (10) pour connexion à un moyen de tube (12) partant de l'adaptateur, et une pointe tubulaire (9) pour pénétrer dans le plongeur lorsque le plongeur (7) est relié à la douille de connexion (5) de manière à relier en écoulement le flacon (2) avec un passage de fluide s'étendant à travers l'adaptateur ;
caractérisé par
un moyen de montage (16 ; 21, 21a) pour monter un dit flacon (2) dans une position sensiblement fixe dans la région de réception de flacon au sein du boîtier (13) ;
un moyen de tube flexible (12) comportant une extrémité couplée à la buse de connexion (10), une première portion (20') retenue dans une position sensiblement fixe relativement au boîtier pour communication avec un moyen de pompage, et une deuxième portion en serpentin (20) entre ladite extrémité et ladite première portion (20') pour permettre le déplacement de l'adaptateur (1, 4) relativement au boîtier sensiblement le long de la région de réception de flacon ; et
un moyen de pompage (14) communiquant avec la première portion (20') du moyen de tube pour pompage de celui-ci par application d'une pression sur l'extérieur du moyen de tube afin de propulser le médicament le long du moyen de tube ;
de manière que, à l'utilisation, la portion de tube (20) permette à l'adaptateur (1, 4) de se déplacer avec le plongeur (7) d'un dit flacon (2) dans le flacon tandis que le médicament est extrait par l'action du moyen de pompage (14) sur le moyen de tube.

6. Appareil selon la revendication 5, dans lequel la pointe tubulaire comprend une extrémité libre pointue et une extrémité de base montées centralement au sein de la douille.

7. Appareil selon la revendication 6, dans lequel la douille comprend un évidement cylindrique (5), la douille comprenant également un taraudage donnant vers l'intérieur pour engager par vissage la partie du plongeur (7) destinée à être reçue dans l'évidement à l'utilisation.

8. Appareil selon l'une quelconque des revendications 5 à 7, dans lequel le boîtier (13) est fixé, avec possibilité de séparation, au moyen de pompage (14).

9. Appareil selon l'une quelconque des revendications 5 à 8, dans lequel le boîtier comprend une plaque de pression sur laquelle la première portion du tube est supportée, la plaque de pression formant une interface entre le boîtier et le moyen de pompage.

10. Appareil selon la revendication 8 ou 9, dans lequel le boîtier (13) comprend un corps creux (15) et une enveloppe externe longitudinale creuse (16) reliée avec possibilité de séparation à une paroi de base (17) du corps creux, et comportant une extrémité fermée et une extrémité ouverte, l'enveloppe externe creuse étant configurée de manière à recevoir et enfermer un dit flacon de médicament.

11. Appareil selon l'une quelconque des revendications 5 à 9, dans lequel le moyen pour monter le flacon comprend une attache (21, 21a).

12. Appareil selon l'une quelconque des revendications 5 à 11, dans lequel le moyen de pompage comprend une pompe péristaltique (14) agissant longitudinalement sur ladite première portion (20') du moyen de tube afin de produire une action de pompage linéaire sur celle-ci.

13. Appareil selon l'une quelconque des revendications 5 à 12, comprenant également un second adaptateur de flacon (1a) destiné à être couplé au plongeur d'un autre flacon de médicament, le tube comprenant également une jonction entre les première et deuxième portions (20', 20) de tube, et une troisième portion de tube (20a) s'étendant de la jonction au second adaptateur pour permettre de dispenser un médicament en parallèle depuis deux flacons.

14. Système pour dispenser des médicaments comprenant un appareil tel que défini selon l'une quelconque des revendications 5 à 13, et un flacon de médicament (2) qui est fermé à une extrémité par un plongeur pénétrable (7) adapté à se déplacer dans le flacon pour diminuer le volume interne d'une quantité correspondant au volume de médicament extrait du flacon, le plongeur étant relié à la douille de connexion de l'adaptateur.

15. Système pour dispenser des médicaments comprenant un appareil tel que défini selon la revendication 13, et des premier et second flacons de médicament (2, 2a), chacun étant fermé à une extrémité par un plongeur pénétrable (7) relié à un des adaptateurs respectifs, chaque plongeur étant adapté à se déplacer dans le flacon respectif pour diminuer le volume interne d'une quantité correspondant au volume de médicament extrait du flacon.
